# EUROPEAN PATENT APPLICATION

(11) **EP 4 559 520 A1**
(43) Date of publication of application: **28.05.2025**
(21) Application number: 24214140.6
(22) Date of filing: 20.11.2024
(51) Int. Cl.: A61P 7/00, A61P 9/00, C07D 487/04

(54) **NOVEL AQP4 INHIBITORS**

(30) Priority: 21.11.2023 EP 23211242
(71) Applicant: Origenis GmbH, 82152 Planegg (DE)
(72) Inventor: The designation of the inventor has not yet been filed
(74) Representative: Forstmeyer, Dietmar

(57) **Abstract**

The present invention relates to novel compounds having the following tautomeric structure of formula (I)

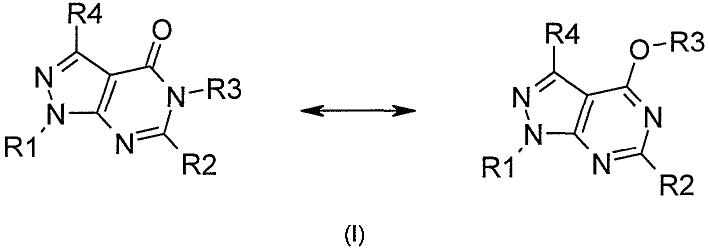

These compounds are aquaporin inhibitors and are useful in the treatment of a variety of diseases, disorders, and conditions that are associated with aberrant activity of one or more aquaporins.

## Description

### FIELD OF THE INVENTION

The present invention relates inter alia to the use of selective aquaporin inhibitors, e.g., of aquaporin-4, e.g., certain 1 H-pyrazolo[3,4-d]pyrimidin-4(5H)-one compounds, for the prophylaxis, treatment and control of aquaporin-mediated conditions, e.g., diseases of water imbalance, for example edema (particularly edema of the brain and spinal cord, e.g., following trauma or ischemic stroke, as well as the edema associated with glioma, meningitis, acute mountain sickness, epileptic seizures, infections, metabolic disorders, hypoxia, water intoxication, hepatic failure, hepatic encephalopathy, diabetic ketoacidosis, abscess, eclampsia, Creutzfeldt-Jakob disease, and lupus cerebritis, as well as edema consequent to microgravity and/or radiation exposure, as well as edema consequent to invasive central nervous system procedures, e.g., neurosurgery, endovascular clot removal, spinal tap, aneurysm repair, or deep brain stimulation, as well as retinal edema), as well as hyponatremia and excess fluid retention, and diseases such as epilepsy, retinal ischemia and other diseases of the eye associated with abnormalities in intraocular pressure and/or tissue hydration, myocardial ischemia, myocardial ischemia/reperfusion injury, myocardial infarction, myocardial hypoxia, congestive heart failure, sepsis, and neuromyelitis optica, as well as migraines and epilepsy.

### BACKGROUND OF THE INVENTION

Aquaporins are cell membrane proteins that act as molecular water channels to mediate the flow of water in and out of the cells. While there is some degree of passive diffusion or osmosis of water across cell membranes, the rapid and selective transport of water in and out of cells involves aquaporins. These water channels selectively conduct water molecules in and out of the cell, while blocking the passage of ions and other solutes, thereby preserving the membrane potential of the cell. Aquaporins are found in virtually all life forms, from bacteria to plants to animals. In humans, they are found in cells throughout the body.

Cerebral edema (CE) is a major contributor to stroke damage, as it can result in increased intracerebral pressure (ICP), a corresponding decrease in cerebral perfusion, and potentially permanent or fatal brain damage. Edema also contributes to CNS damage in, for example, traumatic brain and spinal cord injury, glioma, meningitis, acute mountain sickness, epileptic seizures, infections, metabolic disorders, hypoxia, water intoxication, hepatic failure, hepatic encephalopathy, diabetic ketoacidosis, abscess, eclampsia, Creutzfeldt-Jakob disease, and lupus cerebritis. Patients surviving the period of maximal ICP, usually the three days following a stroke or traumatic brain injury, are likely to survive. Unfortunately, only a few treatment options are available for CE, and these are of limited efficacy. Hyponatremia, characterized by serum sodium levels <135 mM, is the most common form of electrolyte imbalance with hospitals nationwide reporting an incidence of 1520%. The associated fluid retention is symptomatic of heart failure (HF), liver cirrhosis, nephrotic disorder, and syndrome of inappropriate antidiuretic hormone secretion (SIADH). Various diuretics are used to treat congestion associated with HF. By inhibiting the Na/K/CI cotransporter in the thick ascending loop of Henle, loop diuretics cause natriuresis by decreasing Na+ and Cl reabsorption from the urine. An alternative therapy for hyponatremia is the use of vasopressin receptor antagonists, which inhibit water reabsorption by inhibiting the vasopressin-induced trafficking of AQP2. Unfortunately, both loop diuretics and vasopressin receptor antagonists act indirectly toward a desired physiological outcome. An ideal drug would block water reabsorption directly, thus minimizing potential side-effects caused by upstream effectors, but no such drugs are currently known.

Epilepsy is a brain disorder characterized by recurrent seizures. Seizures occur because of disturbed brain activity resulting in some degree of temporary brain dysfunction. Seizures may cause uncontrollable shaking and loss of consciousness but, more commonly, a person experiencing a seizure stops moving or becomes unaware of what is happening. Anticonvulsants may be used to treat epilepsy, however anticonvulsants are not effective for all people with epilepsy.

Ischemia is a condition characterized by an interruption or inadequate supply of blood to tissues. Retinal ischemia occurs due to a deficient supply of blood to the retina. Vascular occlusion, glaucoma, and diabetic retinopathy are associated with retinal ischemia and can produce retinal edema and ganglion cell death leading to visual impairment and blindness. AQP4 is expressed in the Muller cells in the retina. Due to relatively ineffectual treatment, retinal ischemia remains a common cause of visual impairment and blindness.

Myocardial ischemia is a condition caused by a blockage or constriction of one more of the coronary arteries, such as can occur with atherosclerotic plaque occlusion or rupture. Myocardial infarction, a heart attack, occurs when myocardial ischemia exceeds a critical threshold and overwhelms myocardial cellular repair mechanisms designed to maintain normal operating function and homeostasis. Myocardial infarction remains a leading cause of morbidity and mortality worldwide. Compounds effective in treating myocardial ischemia, myocardial ischemia/reperfusion injury, myocardial infarction, and congestive heart failure would be useful pharmaceuticals.

Prior to this invention, there have been no known specific, validated inhibitors of aquaporins, for example AQP4 or AQP2. Certain antiepileptic or sulfonamide drugs (e.g., acetylsulfanilamide, acetazolamide, 6-ethoxy-benzothiazole-2-sulfonamide, topiramate, zonisamide, phenytoin, lamotrigine, and sumatriptan) were at one point reported to be possible inhibitors of AQP4, but this later proved to be incorrect (Yang, et al., Bioorganic & Medicinal Chemistry (2008) 16: 7489-7493). Niclosamid and analogues were reported by Aeromics (AU2013259526A1) to be inhibitors of AQP4. The intravenous prodrug of AER-270, AER-271 (SIM0307) entered Phase I clinical trial in 2021 in healthy volunteers.

There is a great need for improved approaches to treating and controlling diseases of water imbalance, such as edema, for example cerebral edema, and water retention and hyponatremia, as well as diseases such as epilepsy, retinal ischemia, myocardial ischemia, myocardial ischemia/reperfusion injury, myocardial infarction, myocardial hypoxia, congestive heart failure, sepsis, and neuromyelitis optica, as well as migraines.

### SUMMARY OF THE INVENTION

The present invention provides one or more compounds having the following tautomeric structure of formula (I) 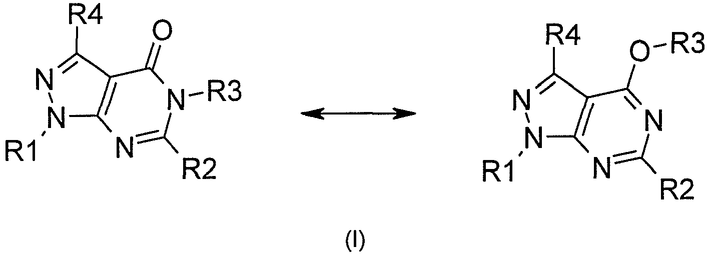 wherein
R1 is H or a C₁₋₆ alkyl group;
R2 is an optionally substituted phenyl group, an optionally substituted naphthyl group or an optionally substituted heteroaryl group containing 5 to 10 ring atoms selected from O, S, N and C;
R3 is H; and
R4 is H or a C₁₋₆ alkyl group;
or a salt thereof.

Preferably, R1 is H or a methyl group; especially H.

Further preferably, R4 is H or a methyl group; especially H.

Moreover preferably, R2 is an optionally substituted phenyl group.

Further preferably, R2 is an optionally substituted heteroaryl group containing one ring and 5 or 6 ring atoms selected from O, S, N and C.

Moreover preferably, R2 is an optionally substituted pyridinyl group; especially a 3-pyridinyl group.

Further preferably, R2 is an optionally substituted heteroaryl group containing two annellated rings and 9 or 10 ring atoms selected from O, S, N and C.

The most preferred compounds of the present invention are the compounds disclosed in the examples, or a salt thereof.

It is further preferred to combine the preferred embodiments of the present invention in any desired manner (e.g., any embodiment for R1 may be combined with any embodiment of R2).

The expression alkyl refers to a saturated, straight-chain or branched hydrocarbon group that contains from 1 to 20 carbon atoms, preferably from 1 to 10 carbon atoms, especially from 1 to 6 (e.g. 1, 2, 3 or 4) carbon atoms, for example a methyl (Me, CH₃), ethyl (Et), n-propyl (nPr), *iso-*propyl (iPr), n-butyl (nBu), iso-butyl (iBu), sec-butyl (sBu), tert-butyl (tBu), n-pentyl, iso-pentyl, n-hexyl, 2,2-dimethylbutyl or n-octyl group.

The expression C₁₋₆ alkyl refers to a saturated, straight-chain or branched hydrocarbon group that contains from 1 to 6 carbon atoms. The expression C₁₋₄ alkyl refers to a saturated, straight-chain or branched hydrocarbon group that contains from 1 to 4 carbon atoms. Examples are a methyl, ethyl, n-propyl, iso-propyl, n-butyl, iso-butyl, sec-butyl or tert-butyl group.

The expressions alkenyl and alkynyl refer to at least partially unsaturated, straight-chain or branched hydrocarbon groups that contain from 2 to 20 carbon atoms, preferably from 2 to 15 carbon atoms, especially from 2 to 10 (e.g., 2, 3 or 4) carbon atoms, for example an ethenyl (vinyl), propenyl (allyl), iso-propenyl, butenyl, ethynyl (acetylenyl), propynyl (e.g., propargyl), butynyl, isoprenyl or hex-2-enyl group. Preferably, alkenyl groups have one or two (especially preferably one) double bond(s), and alkynyl groups have one or two (especially preferably one) triple bond(s).

Furthermore, the terms alkyl, alkenyl and alkynyl refer to groups in which one or more hydrogen atoms have been replaced by a halogen atom (preferably F or CI) such as, for example, a 2,2,2-trichloroethyl or a trifluoromethyl group.

The expression heteroalkyl refers to an alkyl, alkenyl or alkynyl group in which one or more (preferably 1 to 8; especially preferably 1, 2, 3 or 4) carbon atoms have been replaced by an oxygen, nitrogen, phosphorus, boron, selenium, silicon or sulfur atom (preferably by an oxygen, sulfur or nitrogen atom) or by a SO or a SO₂ group. The expression heteroalkyl furthermore refers to a carboxylic acid or to a group derived from a carboxylic acid, such as, for example, acyl, acylalkyl, alkoxycarbonyl, acyloxy, acyloxyalkyl, carboxyalkylamide or alkoxycarbonyloxy. Furthermore, the term heteroalkyl refers to groups in which one or more hydrogen atoms have been replaced by a halogen atom (preferably F or Cl).

Preferably, a heteroalkyl group contains from 1 to 12 carbon atoms and from 1 to 8 heteroatoms selected from oxygen, nitrogen and sulfur (especially oxygen and nitrogen). Especially preferably, a heteroalkyl group contains from 1 to 6 (e.g. 1, 2, 3 or 4) carbon atoms and 1, 2, 3 or 4 (especially 1, 2 or 3) heteroatoms selected from oxygen, nitrogen and sulfur (especially oxygen and nitrogen). The term C₁-₈ heteroalkyl refers to a heteroalkyl group containing from 1 to 8 carbon atoms and 1, 2, 3, 4 or 5 heteroatoms selected from O, S and/or N (especially O and/or N). The term C₁₋₆ heteroalkyl refers to a heteroalkyl group containing from 1 to 6 carbon atoms and 1, 2, 3 or 4 heteroatoms selected from O, S and/or N (especially O and/or N). The term C₁-₄ heteroalkyl refers to a heteroalkyl group containing from 1 to 4 carbon atoms and 1, 2 or 3 heteroatoms selected from O, S and/or N (especially O and/or N).

Examples of heteroalkyl groups are groups of formulae: R^{a}-O-Y^{a}-, R^{a}-S-Y^{a}-, R^{a}-SO-Y^{a}-, R^{a}-SO₂-Y^{a}-, R^{a}-N(R^{b})-SO₂-Y^{a}-, R^{a}-SO₂-N(R^{b})-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}-, R^{a}-N(R^{b})-CO-Y^{a}-, R^{a}-O-CO-N(R^{b})-Y^{a}-, R^{a}-N(R^{b})-CO-O-Y^{a}-, -Y^{a}-CN, R^{a}-N(R^{b})-CO-N(R^{c})-Y^{a}-, R^{a}-O-CO-O-Y^{a}-, R^{a}-N(R^{b})-C(=NR^{d})-N(R^{c})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y^{a}-, R^{a}-O-CS-N(R^{b})-Y^{a}-, R^{a}-N(R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}-, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-, wherein R^{a} being a hydrogen atom, a C₁-C₆ alkyl, a C₂-C₆ alkenyl or a C₂-C₆ alkynyl group; R^{b} being a hydrogen atom, a C₁-C₆ alkyl, a C₂-C₆ alkenyl or a C₂-C₆ alkynyl group; R^{c} being a hydrogen atom, a C₁-C₆ alkyl, a C₂-C₆ alkenyl or a C₂-C₆ alkynyl group; R^{d} being a hydrogen atom, a C₁-C₆ alkyl, a C₂-C₆ alkenyl or a C₂-C₆ alkynyl group and Y^{a} being a bond, a C₁-C₆ alkylene, a C₂-C₆ alkenylene or a C₂-C₆ alkynylene group, wherein each heteroalkyl group contains at least one carbon atom and one or more hydrogen atoms may be replaced by fluorine or chlorine atoms.

Specific examples of heteroalkyl groups are methoxy, trifluoromethoxy, ethoxy, n-propyloxy, iso-propyloxy, n-butoxy, tert-butyloxy, methoxymethyl, -CH₂CH₂OH, -CH₂OH, -SO₂Me, -NHAc, - C(CH₃)₂CN, -COO^{t}Bu, methoxyethyl, ethoxymethyl, 1-methoxyethyl, 1-ethoxyethyl, 2-methoxyethyl or 2-ethoxyethyl, methylamino, ethylamino, propylamino, isopropylamino, dimethylamino, diethylamino, isopropylethylamino, methylamino methyl, ethylamino methyl, diisopropylamino ethyl, methylthio, ethylthio, isopropylthio, enol ether, dimethylamino methyl, dimethylamino ethyl, acetyl, propionyl, butyryloxy, acetyloxy, methoxycarbonyl, ethoxycarbonyl, propionyloxy, acetylamino or propionylamino, carboxymethyl, carboxyethyl or carboxypropyl, N-ethyl-*N*-methylcarbamoyl or *N*-methylcarbamoyl. Further examples of heteroalkyl groups are nitrile (-CN), isonitrile, cyanate, thiocyanate, isocyanate, isothiocyanate and alkylnitrile groups.

The expression cycloalkyl refers to a saturated or partially unsaturated (for example, a cycloalkenyl group) cyclic group that contains one or more rings (preferably 1 or 2), and contains from 3 to 14 ring carbon atoms, preferably from 3 to 10 (especially 3, 4, 5, 6 or 7) ring carbon atoms. The expression cycloalkyl refers furthermore to groups in which one or more hydrogen atoms have been replaced by fluorine, chlorine, bromine or iodine atoms or by OH, =O, SH, =S, NH₂, =NH, N₃ or NO₂ groups, thus, for example, cyclic ketones such as, for example, cyclohexanone, 2-cyclohexenone or cyclopentanone. Further specific examples of cycloalkyl groups are a cyclopropyl, cyclobutyl, cyclopentyl, spiro[4,5]decanyl, norbornyl, cyclohexyl, cyclopentenyl, cyclohexadienyl, decalinyl, bicyclo[4.3.0]nonyl, tetraline, cyclopentylcyclohexyl, fluorocyclohexyl or cyclohex-2-enyl group. Preferably, the expression cycloalkyl refers to a saturated cyclic group that contains one or more rings (preferably 1 or 2), and contains from 3 to 14 ring carbon atoms, preferably from 3 to 10 (especially 3, 4, 5, 6 or 7) ring carbon atoms.

The expression heterocycloalkyl refers to a cycloalkyl group as defined above in which one or more (preferably 1, 2 or 3) ring carbon atoms have been replaced by an oxygen, nitrogen, silicon, selenium, phosphorus or sulfur atom (preferably by an oxygen, sulfur or nitrogen atom) or a SO group or a SO₂ group. A heterocycloalkyl group has preferably 1 or 2 ring(s) and 3 to 10 (especially 3, 4, 5, 6 or 7) ring atoms (preferably selected from C, O, N and S). The expression heterocycloalkyl refers furthermore to groups that are substituted by fluorine, chlorine, bromine or iodine atoms or by OH, =O, SH, =S, NH₂, =NH, N₃ or NO₂ groups. Examples are a piperidyl, prolinyl, imidazolidinyl, piperazinyl, morpholinyl (e.g. -N(CH₂CH₂)₂O), urotropinyl, pyrrolidinyl, tetrahydrothiophenyl, tetrahydropyranyl, tetrahydrofuryl or 2-pyrazolinyl group and also lactames, lactones, cyclic imides and cyclic anhydrides.

The expression alkylcycloalkyl refers to groups that contain both cycloalkyl and alkyl, alkenyl or alkynyl groups in accordance with the above definitions, for example alkylcycloalkyl, cycloalkylalkyl, alkylcycloalkenyl, alkenylcycloalkyl and alkynylcycloalkyl groups. An alkylcycloalkyl group preferably contains a cycloalkyl group that contains one or two rings and from 3 to 10 (especially 3, 4, 5, 6 or 7) ring carbon atoms, and one or two alkyl, alkenyl or alkynyl groups (especially alkyl groups) having 1 or 2 to 6 carbon atoms.

The expression heteroalkylcycloalkyl refers to alkylcycloalkyl groups as defined above in which one or more (preferably 1, 2 or 3) carbon atoms have been replaced by an oxygen, nitrogen, silicon, selenium, phosphorus or sulfur atom (preferably by an oxygen, sulfur or nitrogen atom) or a SO group or a SO₂ group. A heteroalkylcycloalkyl group preferably contains 1 or 2 rings having from 3 to 10 (especially 3, 4, 5, 6 or 7) ring atoms, and one or two alkyl, alkenyl, alkynyl or heteroalkyl groups (especially alkyl or heteroalkyl groups) having from 1 or 2 to 6 carbon atoms. Examples of such groups are alkylheterocycloalkyl, alkylheterocycloalkenyl, alkenylheterocycloalkyl, alkynylheterocycloalkyl, heteroalkylcycloalkyl, heteroalkylheterocycloalkyl and heteroalkylheterocycloalkenyl, the cyclic groups being saturated or mono-, di- or tri-unsaturated.

The expression aryl refers to an aromatic group that contains one or more rings and from 6 to 14 ring carbon atoms, preferably from 6 to 10 (especially 6) ring carbon atoms. The expression aryl refers furthermore to groups that are substituted by fluorine, chlorine, bromine or iodine atoms or by OH, SH, NH₂, N₃ or NO₂ groups. Examples are the phenyl (Ph), naphthyl, biphenyl, 2-fluorophenyl, anilinyl, 3-nitrophenyl or 4-hydroxyphenyl group.

The expression heteroaryl refers to an aromatic group that contains one or more rings and from 5 to 14 ring atoms, preferably from 5 to 10 (especially 5 or 6 or 9 or 10) ring atoms, comprising one or more (preferably 1, 2, 3 or 4) oxygen, nitrogen, phosphorus or sulfur ring atoms (preferably O, S or N). The expression heteroaryl refers furthermore to groups that are substituted by fluorine, chlorine, bromine or iodine atoms or by OH, SH, N₃, NH₂ or NO₂ groups. Examples are pyridyl (e.g. 4-pyridyl), imidazolyl (e.g. 2-imidazolyl), phenylpyrrolyl (e.g. 3-phenylpyrrolyl), thiazolyl, isothiazolyl, 1,2,3-triazolyl, 1,2,4-triazolyl, oxadiazolyl, thiadiazolyl, indolyl, indazolyl, tetrazolyl, pyrazinyl, pyrimidinyl, pyridazinyl, 4-hydroxypyridyl (4-pyridonyl), 3,4-hydroxypyridyl (3,4-pyridonyl), oxazolyl, isoxazolyl, triazolyl, tetrazolyl, isoxazolyl, indazolyl, indolyl, benzimidazolyl, benzoxazolyl, benzisoxazolyl, benzthiazolyl, pyridazinyl, quinolinyl, isoquinolinyl, pyrrolyl, purinyl, carbazolyl, acridinyl, pyrimidyl, 2,3'-bifuryl, 2-oxo-1,2-dihydropyridin-3-yl, pyrazolyl (e.g. 3-pyrazolyl) and isoquinolinyl groups.

The expression aralkyl refers to groups containing both aryl and also alkyl, alkenyl, alkynyl and/or cycloalkyl groups in accordance with the above definitions, such as, for example, arylalkyl, arylalkenyl, arylalkynyl, arylcycloalkyl, arylcycloalkenyl, alkylarylcycloalkyl and alkylarylcycloalkenyl groups. Specific examples of aralkyls are phenylcyclopentyl, cyclohexylphenyl as well as groups derived from toluene, xylene, mesitylene, styrene, benzyl chloride, o-fluorotoluene, 1H-indene, tetraline, dihydronaphthalene, indanone, cumene, fluorene and indane. An aralkyl group preferably contains one or two aromatic ring systems (especially 1 or 2 rings), each containing from 6 to 10 carbon atoms and one or two alkyl, alkenyl and/or alkynyl groups containing from 1 or 2 to 6 carbon atoms and/or one or two cycloalkyl group containing 3, 4, 5, 6 or 7 ring carbon atoms.

The expression heteroaralkyl refers to groups containing both aryl and/or heteroaryl groups and also alkyl, alkenyl, alkynyl and/or heteroalkyl and/or cycloalkyl and/or heterocycloalkyl groups in accordance with the above definitions. A heteroaralkyl group preferably contains one or two aromatic ring systems (especially 1 or 2 rings), each containing from 5 or 6 to 9 or 10 ring atoms (preferably selected from C, N, O and S) and one or two alkyl, alkenyl and/or alkynyl groups containing 1 or 2 to 6 carbon atoms and/or one or two heteroalkyl groups containing 1 to 6 carbon atoms and 1, 2 or 3 heteroatoms selected from O, S and N and/or one or two cycloalkyl groups each containing 3, 4, 5, 6 or 7 ring carbon atoms and/or one or two heterocycloalkyl groups, each containing 3, 4, 5, 6 or 7 ring atoms comprising 1, 2, 3 or 4 oxygen, sulfur or nitrogen atoms.

Examples are arylheteroalkyl, arylheterocycloalkyl, arylheterocycloalkenyl, arylalkylheterocycloalkyl, arylalkenylheterocycloalkyl, arylalkynylheterocycloalkyl, arylalkylheterocycloalkenyl, heteroarylalkyl, heteroarylalkenyl, heteroarylalkynyl, heteroarylheteroalkyl, heteroarylcycloalkyl, heteroarylcycloalkenyl, heteroaryl-heterocycloalkyl, heteroarylheterocycloalkenyl, heteroarylalkylcycloalkyl, heteroaryl-alkylheterocycloalkenyl, heteroarylheteroalkylcycloalkyl, heteroarylheteroalkyl-cycloalkenyl and heteroarylheteroalkylheterocycloalkyl groups, the cyclic groups being saturated or mono-, di- or tri-unsaturated. Specific examples are a tetrahydroisoquinolinyl, benzoyl, phthalidyl, 2- or 3-ethylindolyl, 4-methylpyridino, 2-, 3- or 4-methoxyphenyl, 4-ethoxyphenyl, 2-, 3- or 4-carboxyphenylalkyl group.

As already stated above, the expressions cycloalkyl, heterocycloalkyl, alkylcycloalkyl, heteroalkylcycloalkyl, aryl, heteroaryl, aralkyl and heteroaralkyl also refer to groups that are substituted by fluorine, chlorine, bromine or iodine atoms or by OH, =O, SH, =S, NH₂, =NH, N₃ or NO₂ groups.

According to a preferred embodiment, all alkyl, alkenyl, alkynyl, heteroalkyl, cycloalkyl, heterocycloalkyl, alkylcycloalkyl, heteroalkylcycloalkyl, aryl, heteroaryl, aralkyl and heteroaralkyl groups described herein may optionally be substituted.

The term halogen refers to F, CI, Br or I.

When an aryl, heteroaryl, cycloalkyl, alkylcycloalkyl, heteroalkylcycloalkyl, heterocycloalkyl, aralkyl or heteroaralkyl group contains more than one ring, these rings may be bonded to each other via a single or double bond or these rings may be annulated or fused or bridged.

The suffix "-ene" like e.g. in "phenylene" refers to the corresponding divalent group.

The term "optionally substituted" refers to a group which is unsubstituted or substituted by one or more (especially by one, two or three; preferably by one or two) substituents.

If a group comprises more than one substituent, these substituents are independently selected, i.e. they may be the same or different.

If a group is substituted by a cyclic group, such as e.g., a cycloalkyl group or a heterocycloalkyl group, or an aryl group, or a heteroaryl group, this cyclic group may be bonded to said group via a single or double bond or this cyclic group may be annulated or fused to said group.

Specific examples for substituents are fluorine, chlorine, bromine and iodine and OH, =O, SH, NH₂, -SO₃H, -SO₂NH₂, -COOH, -COOMe, -COMe (Ac), -NHSO₂Me, -SO₂NMe₂, -CH₂NH₂, -NHAc, -SO₂Me, -COO^{t}Bu, NMe₂, Me, -N(CH₂CH₂)₂NMe, -N(CH₂CH₂)₂O, -CONH₂, -CN, -NHCONH₂, - NHC(NH)NH₂, -NOHCH₃, -N₃ and -NO₂ groups.

Further examples of substituents are C₁-C₁₀ alkyl, C₂-C₁₀ alkenyl, C₂-C₁₀ alkynyl, C₁-C₁₀ heteroalkyl, C₃-C₁₈ cycloalkyl, C₁-C₁₇ heterocycloalkyl, C₄-C₂₀ alkylcycloalkyl, C₁-C₁₉ heteroalkylcycloalkyl, C₆-C₁₈ aryl, C₁-C₁₇ heteroaryl, C₇-C₂₀ aralkyl and C₁-C₁₉ heteroaralkyl groups; especially C₁-C₆ alkyl, C₂-C₆ alkenyl, C₂-C₆ alkynyl, C₁-C₆ heteroalkyl, C₃-C₁₀ cycloalkyl, C₁-C₉ heterocycloalkyl, C₄-C₁₂ alkylcycloalkyl, C₁-C₁₁ heteroalkylcycloalkyl, C₆-C₁₀ aryl, C₁-C₉ heteroaryl, C₇-C₁₂ aralkyl and C₁-C₁₁ heteroaralkyl groups, further preferably C₁-C₆ alkyl and C₁-C₆ heteroalkyl groups.

Preferred substituents are halogen atoms (e.g., F, Cl, Br) and -OH, =O, -NH₂, -CN, C₁-C₆ alkyl and C₁-C₆ heteroalkyl groups as well as C₃-C₇ cycloalkyl groups and heterocycloalkyl groups containing from 3 to 7 ring atoms selected from C, N and O.

Further preferred substituents are halogen atoms (e.g. F, Cl, Br) and -OH, =O, -NH₂, -CN, -C₁₋₄ alkyl (e.g. -Me, -Et, -nPr, -iPr, -nBu, -iBu, -tBu, -CH₂CH₂F, CH₂CHF₂, -CH₂CF₃ and -CF₃), -O-C₁₋₄ alkyl (e.g. -OMe, -OEt, -O-nPr, -O-iPr, -O-nBu, -O-iBu and -O-tBu), -NHC₁₋₆ alkyl (e.g., - NH(CH₂)₂F and -NH(CH₂)₃F), -NH(CH₂)₂OH, -NH(CH₂)₃OH, -NH(CH₂)₂OMe, -NH(CH₂)₃OMe, - N(Me)(CH₂)₂OH, -N(Me)(CH₂)₃OH, -N(Me)(CH₂)₂OMe, -N(Me)(CH₂)₃OMe, -N(C₁₋₆ alkyl)₂, - C(CH₃)₂CN, -CONH-C₁₋₄ alkyl (e.g. -CONHCH₂CF₃, -CONHEt, -CONH^{t}Bu), -COOH, -COOMe, - COMe, -CH₂CH₂CH=CH₂, pyrrolidinyl, cyclopropyl and -CH₂-cyclopropyl groups.

The therapeutic use of compounds according to formula (I), their pharmacologically acceptable salts, solvates and hydrates, respectively, as well as formulations and pharmaceutical compositions also lie within the scope of the present invention.

The present invention further provides pharmaceutical compositions comprising one or more compounds of formula (I) or a salt thereof as defined herein or a pharmaceutically acceptable ester, prodrug, hydrate or solvate thereof, optionally in combination with a pharmaceutically acceptable carrier and/or adjuvant.

It is a further object of the present invention to provide a compound of formula (I) as defined herein or a pharmaceutical composition as defined herein for the preparation of a medicament for the treatment of one or more diseases specified herein.

The present invention especially provides the use of selective aquaporin inhibitors, e.g., of aquaporin-4 for the prophylaxis, treatment and control of aquaporin-mediated conditions, e.g., diseases of water imbalance, for example edema (particularly edema of the brain and spinal cord, e.g., following trauma or ischemic stroke, as well as the edema associated with glioma, meningitis, acute mountain sickness, epileptic seizures, infections, metabolic disorders, water intoxication, hepatic failure, hepatic encephalopathy, diabetic ketoacidosis, abscess, eclampsia, Creutzfeldt-Jakob disease, and lupus cerebritis, as well as the edema consequent to microgravity and/or radiation exposure, as well as edema consequent to invasive central nervous system procedures, e.g., neurosurgery, endovascular clot removal, spinal tap, aneurysm repair, or deep brain stimulation, as well as retinal edema, as well as brain swelling consequent to cardiac arrest, e.g., related to the development of the metabolic acidosis (e.g. lactic acidosis) due to hypoxia before the resuscitation period), as well as hyponatremia and excess fluid retention, as well as diseases such as epilepsy, retinal ischemia and other diseases of the eye associated with abnormalities in intraocular pressure or tissue hydration, myocardial ischemia, myocardial ischemia/reperfusion injury, myocardial infarction, myocardial hypoxia, congestive heart failure, sepsis, and neuromyelitis optica, as well as migraines.

The present invention provides, inter alia, methods of treating or controlling a disease or condition mediated by an aquaporin, e.g., diseases or conditions of water imbalance and other diseases, for example, edema of the brain or spinal cord, e.g., cerebral edema, e.g. cerebral edema consequent to head trauma, ischemic stroke, glioma, meningitis, acute mountain sickness, epileptic seizures, infections, metabolic disorders, hypoxia (including general systemic hypoxia and hypoxia due to cardiac arrest), water intoxication, hepatic failure, hepatic encephalopathy, diabetic ketoacidosis, abscess, eclampsia, Creutzfeldt-Jakob disease, lupus cerebritis, or invasive central nervous system procedures, e.g., neurosurgery, endovascular clot removal, spinal tap, aneurysm repair, or deep brain stimulation or, e.g., spinal cord edema consequent to spinal cord trauma, e.g., spinal cord compression; or cerebral and/or optical nerve edema consequent to microgravity and/or radiation exposure; or retinal edema; or hyponatremia or excessive fluid retention, e.g., consequent to heart failure (HF), liver cirrhosis, nephrotic disorder, or syndrome of inappropriate antidiuretic hormone secretion (SIADH); or epilepsy, retinal ischemia or other diseases of the eye associated with abnormalities in intraocular pressure and/or tissue hydration, myocardial ischemia, myocardial ischemia/reperfusion injury, myocardial infarction, myocardial hypoxia, congestive heart failure, sepsis, or neuromyelitis optica; or migraines, comprising administering to a patient in need thereof an effective amount of an aquaporin inhibitor of the 1 H-pyrazolo[3,4-d]pyrimidin-4(5H)-one class of formula (I).

A therapeutically effective amount of a compound in accordance with this invention means an amount of compound that is effective to prevent, alleviate or ameliorate symptoms of a disease or prolong the survival of the subject being treated. Determination of a therapeutically effective amount is within the skill in the art.

The therapeutically effective amount or dosage of a compound according to this invention can vary within wide limits and may be determined in a manner known in the art. Such dosage may be adjusted to the individual requirements in each particular case including the specific compound being administered, the route of administration, the condition being treated, as well as the patient being treated.

The salt of a compound of formula (I) is preferably a pharmacologically acceptable salt. Examples of pharmacologically acceptable salts of sufficiently basic compounds of formula (I) are salts of physiologically acceptable mineral acids like hydrochloric, hydrobromic, sulfuric and phosphoric acid; or salts of organic acids like methanesulfonic, p-toluenesulfonic, lactic, acetic, trifluoroacetic, citric, succinic, fumaric, maleic and salicylic acid. Further, a sufficiently acidic compound of formula (I) may form alkali or earth alkali metal salts, for example sodium, potassium, lithium, calcium or magnesium salts; ammonium salts; or organic base salts, for example methylamine, dimethylamine, trimethylamine, triethylamine, ethylenediamine, ethanolamine, choline hydroxide, meglumin, piperidine, morpholine, tris-(2-hydroxyethyl)amine, lysine or arginine salts; all of which are also further examples of salts of formula (I).

Compounds of formula (I) may be solvated, especially hydrated. The hydratization/hydration may occur during the process of production or as a consequence of the hygroscopic nature of the initially water free compounds of formula (I). The solvates and/or hydrates may e.g. be present in solid or liquid form.

It should be appreciated that certain compounds of formula (I) may have tautomeric forms from which only one might be specifically mentioned or depicted in the following description, different geometrical isomers (which are usually denoted as cis/trans isomers or more generally as (E) and (Z) isomers) or different optical isomers as a result of one or more chiral carbon atoms (which are usually nomenclatured under the Cahn-Ingold-Prelog or R/S system). All these tautomeric forms, geometrical or optical isomers (as well as racemates and diastereomers) and polymorphous forms are included in the invention. Since the compounds of formula (I) may contain asymmetric C-atoms, they may be present either as achiral compounds, mixtures of diastereomers, mixtures of enantiomers or as optically pure compounds. The present invention comprises both all pure enantiomers and all pure diastereomers, and also the mixtures thereof in any mixing ratio.

According to a further embodiment of the present invention, one or more hydrogen atoms of the compounds of the present invention may be replaced by deuterium. Deuterium modification improves the metabolic properties of a drug with little or no change in its intrinsic pharmacology. Deuterium substitution at specific molecular positions improves metabolic stability, reduces formation of toxic metabolites and/or increases the formation of desired active metabolites. Accordingly, the present invention also encompasses the partially and fully deuterated compounds of formula (I). The term hydrogen also encompasses deuterium.

The present invention also relates to pro-drugs which are composed of a compound of formula (I) and at least one pharmacologically acceptable protective group which will be cleaved off under physiological conditions, such as an alkoxy-, arylalkyloxy-, acyl-, acyloxymethyl group (e.g. pivaloyloxymethyl), an 2-alkyl-, 2-aryl- or 2-arylalkyl-oxycarbonyl-2-alkylidene ethyl group or an acyloxy group as defined herein, e.g. ethoxy, benzyloxy, acetyl or acetyloxy or, especially for a compound of formula (I), carrying a hydroxy group (-OH): a sulfate, a phosphate (-OPO₃ or - OCH₂OPO₃) or an ester of an amino acid. Especially preferred are pro-drugs of the hydroxy group of a compound of formula (I).

As used herein, the term pharmaceutically acceptable ester especially refers to esters which hydrolyze in vivo and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters include, but are not limited to, formates, acetates, propionates, butyrates, acrylates and ethylsuccinates.

Preferably, the present invention also relates to a prodrug, a biohydrolyzable ester, a biohydrolyzable amide, a polymorph, tautomer, stereoisomer, metabolite, N-oxide, biohydrolyzable carbamate, biohydrolyzable ether, physiologically functional derivative, atropisomer, or in vivo-hydrolysable precursor, diastereomer or mixture of diastereomers, chemically protected form, affinity reagent, complex, chelate and a stereoisomer of the compounds of formula (I).

As mentioned above, therapeutically useful agents that contain compounds of formula (I), their solvates, salts or formulations are also comprised in the scope of the present invention. In general, compounds of formula (I) will be administered by using the known and acceptable modes known in the art, either alone or in combination with any other therapeutic agent.

For oral administration such therapeutically useful agents can be administered by one of the following routes: oral, e.g. as tablets, dragees, coated tablets, pills, semisolids, soft or hard capsules, for example soft and hard gelatine capsules, aqueous or oily solutions, emulsions, suspensions or syrups, parenteral including intravenous, intramuscular and subcutaneous injection, e.g. as an injectable solution or suspension, rectal as suppositories, by inhalation or insufflation, e.g. as a powder formulation, as microcrystals or as a spray (e.g. liquid aerosol), transdermal, for example via an transdermal delivery system (TDS) such as a plaster containing the active ingredient or intranasal. For the production of such tablets, pills, semisolids, coated tablets, dragees and hard, e.g. gelatine, capsules the therapeutically useful product may be mixed with pharmaceutically inert, inorganic or organic excipients as are e.g. lactose, sucrose, glucose, gelatine, malt, silica gel, starch or derivatives thereof, talc, stearinic acid or their salts, dried skim milk, and the like. For the production of soft capsules, one may use excipients as are e.g. vegetable, petroleum, animal or synthetic oils, wax, fat, polyols. For the production of liquid solutions, emulsions or suspensions or syrups one may use as excipients e.g. water, alcohols, aqueous saline, aqueous dextrose, polyols, glycerin, lipids, phospholipids, cyclodextrins, vegetable, petroleum, animal or synthetic oils. Especially preferred are lipids and more preferred are phospholipids (preferred of natural origin; especially preferred with a particle size between 300 to 350 nm) preferred in phosphate buffered saline (pH = 7 to 8, preferred 7.4). For suppositories one may use excipients as are e.g. vegetable, petroleum, animal or synthetic oils, wax, fat and polyols. For aerosol formulations one may use compressed gases suitable for this purpose, as are e.g. oxygen, nitrogen and carbon dioxide. The pharmaceutically useful agents may also contain additives for conservation, stabilization, e.g. UV stabilizers, emulsifiers, sweetener, aromatizers, salts to change the osmotic pressure, buffers, coating additives and antioxidants.

In general, in the case of oral or parenteral administration to adult humans weighing approximately 80 kg, a daily dosage of about 10 mg to about 10,000 mg, preferably from about 20 mg to about 1,000 mg, should be appropriate, although the upper limit may be exceeded when indicated. The daily dosage can be administered as a single dose or in divided doses, or for parenteral administration, it may be given as continuous infusion or subcutaneous injection.

According to a moreover preferred embodiment, the present invention provides a method for treating one or more diseases specified herein which comprises administering to a subject in need of such treatment a therapeutically effective amount of a compound of formula (I), or a pharmaceutically acceptable salt thereof.

According to a further preferred embodiment, the present invention provides a method for treating one or more diseases specified herein which comprises administering to a subject in need of such treatment a pharmaceutical composition comprising a compound of formula (I), or a pharmaceutically acceptable salt thereof.

### EXAMPLES

### BIOLOGY

The inhibition of AQP4 was evaluated in *Xenopus laevis* oocytes.

**RNA preparation and heterologous expression in *Xenopus laevis* oocytes** - The most prominent isoform of AQP4 in the brain, called M23 due to its translation initiation site at methionine at position 23 in the primary structure of AQP4 ( M. Amiry-Moghaddam, O. P. Ottersen, The molecular basis of water transport in the brain. Nat Rev Neurosci 4, 991-1001 (2003)), was employed for these experiments. cDNA encoding rat AQP4 was subcloned into an oocyte expression vectors pXOOM, linearized downstream from the poly-A segment, and *in vitro* transcribed using T7 mMessage machine according to manufacturer's instructions (Ambion, Austin, TX). cRNA was extracted with MEGAclear (Ambion, Austin, TX) and micro-injected into defolliculated *Xenopus laevis* oocytes (EcoCyte Bioscience (Germany)): 10 ng RNA was injected per oocyte and the oocytes kept in Kulori medium ((in mM): 90 NaCl, 1 KCI, 1 CaCl₂, 1 MgCl₂, 5 HEPES (pH 7.4)) for 3-4 days at 19°C prior to experiments.

**Volume recordings of oocytes** - Oocytes were placed in an experimental recording chamber, perfused with various solutions, and volume measurements were performed as previously described ( T. Zeuthen, E. Zeuthen, N. Macaulay, Water transport by GLUT2 expressed in Xenopus laevis oocytes. J Physiol 579, 345-361 (2007)). Briefly, the oocytes were placed in a small chamber with a glass bottom and perfused with solutions of interest. The volume of the oocytes was viewed from below via a long-distance objective, and micrographs were captured continuously with a high-resolution recording system (with a high signal to noise ratio) based on a CCD camera at a rate of 25 images/s ( T. Zeuthen, B. Belhage, E. Zeuthen, Water transport by Na+-coupled cotransporters of glucose (SGLT1) and of iodide (NIS). The dependence of substrate size studied at high resolution. J Physiol 570, 485-499 (2006)). The control perfusion solution consisted of (in mM): 50 NaCl, 2 KCI, 1 MgCl₂, 1 CaCl₂, 10 HEPES, 100 mM mannitol (Tris buffered pH 7.4, 220 mOsm). Hyposmotic solutions were subsequently made by the removal of mannitol (Δ100 mM mannitol) with resulting osmolarity of 120 mOsm. The osmotic water permeability was determined by Lₚ = (Jᵥ)/(A × Δπ × V_{w}), where *Jᵥ* is the initial water flux during an osmotic challenge, A is the membrane surface area (nine times the apparent area due to membrane folding ( G. A. Zampighi et al., A method for determining the unitary functional capacity of cloned channels and transporters expressed in Xenopus laevis oocytes. J Membr Biol 148, 65-78 (1995)), *Δπ* is the osmotic challenge, and *V_{w}* is the partial molal volume of water (18 cm³/mol). Osmolarities of all solutions were verified with an accuracy of 1 mOsm with an osmometer Type 15 (Löser Messtechnik, Berlin, Germany). The compounds used were dissolved in DMSO (controls exposed to vehicle (DMSO) only) and their effect on volume changes were recorded after 10 min of treatment with the oocytes serving as own controls or after long-term application where the oocytes were incubated at 19 °C for 60 min. Determination of compound-mediated effects was carried out in a researcher-blinded fashion.

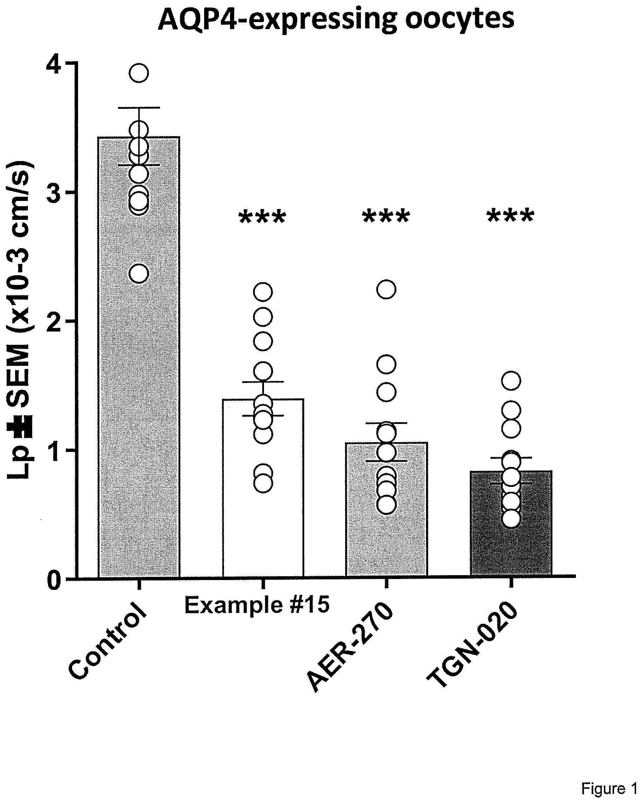

**Data presentation and statistics** - All data are given as mean ± SEM. Statistical tests were performed by use of GraphPad Prism 8.4.3 (GraphPad Software Inc., La Jolla, CA, USA). Statistical significance was tested with Student's t-test or one-way ANOVA as stated in the figure legends (Figure 1). *P* values < 0.05 were considered statistically significant. The number of experiments (n) corresponds to independent measurements from two-three different oocyte preparations. The equation y = 100/(1 + 10Λ((logIC₅₀ - x) × Hill slope)) was used to fit the IC₅₀ curves for each individual experiment, which are shown as summarized.

### General Synthesis methods

The following methods were used in synthesis of the compounds described herein.

Flash chromatography: Flash chromatography was performed on a Biotage Isolera(R) or Selekt^{®} system using SNAP or SFÄR silica cartridges and ethyl acetate/cyclohexane/methanol or dichloromethane/methanol gradients as eluent.

Microwave conditions: Reactions under microwave conditions are performed in a Biotage initiator(R) microwave system.

SEMI prep reversed phase chromatography: The following instrumentation was used for SEMlprep reversed phase chromatography: 2x Varian PrepStar SD-1, 1x Dionex P580 Pump 1 Channel(MakeUP I), 1x Dionex AXP-MS (MakeUP II), 1x Dionex MSQ, 1x Dionex UVD 340V - Prep Flow Cell, Gilson 215 Liquid Handler, SunFire Prep C18 OBD 5 µm, 19x50 mm column, 1x G7159B 1290 Infinity II Preparative Open-Bed Sampler/Collector, 1x G7161B 1290 Infinity II Preparative Binary Pump, 1x G7111B 1260 Infinity II Quaternary Pump (Modifier), 1x G7111B 1260 Infinity II Quaternary Pump (Analyltic / MakeUp), 1x G7165A 1260 Infinity II Multiple Wavelength Detector incl. Flow Cell (Product# G1315-60022, Serial# DE185H6157, Path Length 10.00 mm, Volume 13.00 µl), 1x G7170B 1290 Infinity II MS Flow Modulator, 1x G6125B MSD 6100 Series Single Quadrupole incl. G1948B Electrospray Interface, and 3x G1170A 1290 Infinity Valve Drive (14 Ports, 6 Positions Valve Head for Analytic Column Selection; 14 Ports, 6 Positions Valve Head for Preparative Column Selection; 14 Ports, 2 Positions Valve Head for Analytic / Preparative Mode Selection).

Preparative columns: Waters SunFire Prep C18 5 µm OBD 30x100 mm, #186002572, Waters Atlantis T3 Prep 5µm OBD 30x100 mm, #186003702, and Waters XSelect CSH Prep C18 5 µm OBD 30x100 mm, #186005425.

Analytical columns: Waters SunFire C18 2.5 µm 3.0x75mm, #186005636, Waters Atlantis T3 3µm 3.0 x75 mm, #186005653, and Waters XSelect CSH C18 2.5 µm 3.0 × 75mm, #186006106.

Typical chromatography conditions are as follows:
Column flow was 30 mL/min, Solvent A was methanol containing 0.3% acetic acid, and Solvent B was water containing 0.3% acetic acid.

Typical times and relative volumes of Solvent and Solvent B are shown in Table 1.

**Table 1**

| Time (min) | Solv. A | Solv. B |
|---|---|---|
| 0.0 | 30.00 | 70.00 |
| 10.0 | 100.00 | 0.00 |
| 14.0 | 100.00 | 0.00 |
| 14.4 | 30.00 | 70.00 |
| 16.4 | 30.00 | 70.00 |

Typical preparative method: Column flow was 60 mL/min, Solvent A was acetonitrile, and Solvent B was water. Preparation included Modifier Flow: 1.8 mL/min Modifier Flow containing 10% Acetic Acid in Acetonirile/Water 1:1 => resulting 0.3% Acetic Acid in Flow; and 0.5M NH₄Ac/NH₄OH-Buffer (pH 9.2) in Acetonitrile/Water 1:9 => resulting 15 mM Buffer Concentration in Flow. MS MakeUp: 0.9 mL/min 0.05% Acetic Acid in Acetonirile/Water 1:1.

Typical Focused Gradient Timetable for e.g. 59.7% Elution Point is shown in Table 2.

**Table 2**

| Time (min) | Solv. A | Solv. B |
|---|---|---|
| -2.37 | 18.6 | 84.4 |
| 0.00 | 18.6 | 84.4 |
| 1.15 | 18.6 | 84.4 |
| 1.16 | 43.5 | 56.5 |
| 8.46 | 63.5 | 36.5 |
| 8.47 | 100 | 0 |
| 10.77 | 100 | 0 |
| 10.78 | 18.6 | 84.4 |

Typical Analytical Modifier: Column flow was 1 mL/min, Solvent A was acetonitrile, Solvent B was water, and Solvent C was 5% acetic Acid in acetonitrile/water 1:1.

Typical times and relative volumes of Solvent, Solvent B, Solvent C are shown in Table 3.

**Table 3**

| Time (min) | Solv. A | Solv. B | Solv. C |
|---|---|---|---|
| 0 | 2 | 96 | 2 |
| 0.5 | 2 | 96 | 2 |
| 5.5 | 96 | 2 | 2 |
| 5.6 | 98 | 0 | 2 |
| 6.9 | 98 | 0 | 2 |
| 7.0 | 2 | 2 | 2 |

A Mass Spectrometer Detector (API-ES, positive) at UV 220 nm, 254 nm, or 310 nm was used for detection.

Terms and abbreviations used in the Examples are provided in Table 4.

**Table 4**

| |
|---|
| DCM - dichloromethane |
| THF- tetrahydofuran |
| MeOH- methanol |
| celite - Diatomaceous earth, celite (R) CAS 61790-53-2 |
| o.n. - over night |
| r.t. - room temperature |
| eq. - equivalent |
| dioxane - 1,4-dioxane |
| brine - saturated aqueous solution of NaCl |
| h - hour |
| TFA - trifluoroacetic acid |
| Boc - *tert*-Butyloxycarbonyl |
| LiHMDS - Lithium bis(trimethylsilyl)amide CAS 4039-32-1 |
| Pd(PPh₃)₄ - Palladium-tetrakis(triphenylphosphine) CAS 14221-01-3 |
| DIPEA - *N*,*N*-Diisopropylethylamine CAS 7087-68-5 |
| DMF - *N*,*N*-Dimethylformamide CAS 68-12-2 |
| DBAD - Di-*tert*-butyl azodicarboxylate CAS 870-50-8 |
| PPh₃ - Triphenylphosphine CAS 603-35-0 |
| T3P - T3P^{®} 2,4,6-Tripropyl-1,3,5,2,4,6-trioxatriphosphorinane-2,4,6-trioxide CAS 68957-94-8 |
| NaO*t*Bu - Sodium *tert*-butoxide CAS 865-48-5 |
| DDQ - 2,3-Dichloro-5,6-dicyano-1,4-benzoquinone |

The analytical HPLC Methods used in preparation of the compounds are listed in Table 5.

**Table 5**

| Method | col | grad | flow (mL/min) | ms |
|---|---|---|---|---|
| HPLC-MS A | Waters ACQUITY UPLC HSS T3 50x2.1 1.8*µ*m PN: 186003538 | 2%-4.0min->95%-1.0min->95%-0.1min->2%-2.9min->2%; ACN/H₂O + 0.1% HCOOH | 0.6 | ESI positive & negative |
| HPLC-MS B | Waters ACQUITY UPLC CSH C18 50x2.1 1.7*µ*m PN: 186005296 | 5%-4.0min->95%-1.0min->95%-0.1min->5%-2.9min->5%; ACN/H₂O + 0.1% HCOOH | 0.6 | ESI positive & negative |
| HPLC-MS C | Waters ACQUITY UPLC CSH C18 50x2.1 1.7 *µ*m PN:186005296 | 4%-4.0min->96%-1.0min->96%-0.1min->4%-2.9min->4%; ACN/H₂O + 100mM NH₄Ac- | 0.6 | ESI positive |
| HPLC-MS D | Waters CORTECS UPLC C18+ 50x2.1 1.6 *µ*m PN:186007114 | 5%-4.0min->95%-1.0min->95%-0.1min->5%-2.9min->5%; ACN/H₂O + 0.1% HCOOH | 0.6 | ESI positive & negative |
| HPLC-MS E | Waters ACQUITY UPLC CSH C18 50x2.1 1.7pm PN: 186005296 | 5%-0.75min->95%-0.5min->95%-0.05min->5%-1.2min->5%; ACN/H₂O + 0.1% HCOOH | 1.2 | ESI positive |

### General Procedures

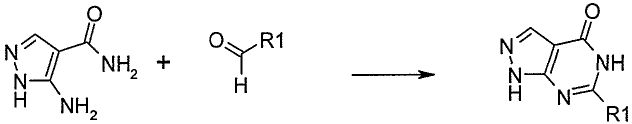

The corresponding pyrazolo amino amide and the aldehyde (1eq.) were dissolved in HOAc (3mUmmol). Then DDQ (0.75 eq.) was added. The reaction mixture was irradiated under microwave conditions at 140°C for 30min. The mixture was filtered, the filtrate diluted with EtOAc (3-5mL/mL HOAc) and cooled to 0°C. The solids were collected by filtration. The combined solids were purified by chromatography.

The pyrazolo amino amide and the aldehydes were commercially available.

The following products were synthesized using this procedure:

| Example | IUPAC name |
|---|---|
| Example#1 | 6-(5-(trifluoromethyl)pyridin-3-yl)-1H-pyrazolo[3,4-d]pyrimidin-4(5H)-one |
| Example#2 | 6-(6-methylpyridin-3-yl)-1H-pyrazolo[3,4-d]pyrimidin-4(5H)-one |
| Example#3 | 6-(pyrazin-2-yl)-1H-pyrazolo[3,4-d]pyrimidin-4(5H)-one |
| Example#4 | 6-(6-methoxypyridin-3-yl)-1H-pyrazolo[3,4-d]pyrimidin-4(5H)-one |
| Example#5 | 6-(6-aminopyridin-3-yl)-1H-pyrazolo[3,4-d]pyrimidin-4(5H)-one |
| Example#6 | 6-(6-(trifluoromethyl)pyridin-3-yl)-1H-pyrazolo[3,4-d]pyrimidin-4(5H)-one |
| Example#7 | 6-(5-fluoropyridin-3-yl)-1H-pyrazolo[3,4-d]pyrimidin-4(5H)-one |
| Example#8 | 3-methyl-6-(pyridin-3-yl)-2H-pyrazolo[3,4-d]pyrimidin-4(5H)-one |
| Example#9 | 1-methyl-6-(pyridin-3-yl)-1H-pyrazolo[3,4-d]pyrimidin-4(2H)-one |
| Example#10 | 6-(pyridazin-4-yl)-1H-pyrazolo[3,4-d]pyrimidin-4(5H)-one |
| Example#11 | 6-(pyridazin-3-yl)-1H-pyrazolo[3,4-d]pyrimidin-4(5H)-one |
| Example#12 | 6-(6-(pyrrolidin-1-yl)pyridin-3-yl)-1H-pyrazolo[3,4-d]pyrimidin-4(5H)-one |
| Example#13 | 6-(pyridin-4-yl)-1H-pyrazolo[3,4-d]pyrimidin-4(5H)-one |
| Example#14 | 6-(pyridin-2-yl)-1H-pyrazolo[3,4-d]pyrimidin-4(5H)-one |
| Example#15 | 6-(pyridin-3-yl)-1H-pyrazolo[3,4-d]pyrimidin-4(5H)-one |
| Example#16 | 6-[3,5-bis(trifluoromethyl)phenyl]-1H-pyrazolo[3,4-d]pyrimidin-4(5H)-one |
| Example#17 | 6-(thiazol-5-yl)-1H-pyrazolo[3,4-d]pyrimidin-4(5H)-one |
| Example#18 | 6-(1,2,3-thiadiazol-4-yl)-1H-pyrazolo[3,4-d]pyrimidin-4(5H)-one |
| Example#19 | 6-(furan-3-yl)-1H-pyrazolo[3,4-d]pyrimidin-4(5H)-one |
| Example#20 | 6-(thiazol-2-yl)-1H-pyrazolo[3,4-d]pyrimidin-4(5H)-one |
| Example#21 | 6-(thiophen-3-yl)-1H-pyrazolo[3,4-d)pyrimidin-4(5H)-one |
| Example#22 | 6-(1-methyl-1H-pyrazol-3-yl)-1H-pyrazolo[3,4-d]pyrimidin-4(5H)-one |
| Example#23 | 6-(1-methyl-1H-pyrazol-4-yl)-1H-pyrazolo[3,4-d]pyrimidin-4(5H)-one |
| Example#24 | 6-(1H-pyrazol-4-yl)-1H-pyrazolo[3,4-djpyrimidin-4(5H)-one |
| Example#25 | 6-(1H-imidazol-4-yl)-1H-pyrazolo[3,4-d]pyrimidin-4(5H)-one |
| Example#26 | 6-(1H-pyrazol-3-yl)-1H-pyrazolo[3,4-d]pyrimidin-4(5H)-one |
| Example#27 | 6-(4H-pyrrolo[3,2-b]pyridin-6-yl)-1H-pyrazolo[3,4-d]pyrimidin-4(5H)-one |
| Example#28 | 6-(quinolin-3-yl)-1H-pyrazolo[3,4-d]pyrimidin-4(5H)-one |
| Example#29 | 6-(2-oxo-1,2-dihydropyridin-3-yl)-2H-pyrazolo[3,4-d]pyrimidin-4(5H)-one |
| Example#30 | 6-(3-methylquinoxalin-2-yl)-2H-pyrazolo[3,4-d]pyrimidin-4(5H)-one |
| Example#31 | 6-(isoxazol-3-yl)-1H-pyrazolo[3,4-d]pyrimidin-4(5H)-one |

### Data of examples:

| Example | Method | r.t.[min] | MH+ | NMR |
|---|---|---|---|---|
| Example#1 | HPLC-MS A | 2.773 | 282.1 | ¹H NMR (599 MHz, dmso-d6) δ 9.51 (d, *J* = 2.1 Hz, 1H), 9.14 (d, *J* = 2.1 Hz, 1H), 8.84 (t, *J* = 2.2 Hz, 1H), 8.23 (s, 1H). |
| Example#2 | HPLC-MS A | 1.968 | 228.0 | |
| Example#3 | HPLC-MS A | 2.177 | 215.0 | ¹H NMR (599 MHz, dmso-d6) δ 9.46 (d, *J =* 1.5 Hz, 1H), 8.84 (d, *J* = 2.5 Hz, 1H), 8.79 - 8.76 (m, 1H), 8.25 (s, 1H). |
| Example#4 | HPLC-MS A | 2.490 | 244.0 | ¹H NMR (599 MHz, dmso-d6) δ 8.88 (dd, *J* = 2.6, 0.7 Hz, 1H), 8.35 (dd, *J* = 8.8, 2.6 Hz, 1H), 8.11 (s, 1H), 6.94 (dd, *J* = 8.7, 0.7 Hz, 1H), 3.90 (s, 3H). |
| Example#5 | HPLC-MS C | 1.387 | 229.0 | ¹H NMR (600 MHz, cdsod) δ 8.62 (d, *J* = 2.2 Hz, 1H), 8.56 (dd, *J* = 9.4, 2.3 Hz, 1H), 8.17 (d, *J =* 12.9 Hz, 1H), 7.92 (dd, *J* = 9.2, 2.1 Hz, 1H), 7.84 - 7.81 (m, 1H), 7.14 (d, *J* = 9.4 Hz, 1H), 7.03 (d, *J* = 9.2 Hz, 1H), 5.37 (s, 1H). |
| Example#6 | HPLC-MS A | 2.776 | 282.1 | |
| Example#7 | HPLC-MS A | 2.262 | 232.0 | ¹H NMR (599 MHz, dmso-d6) δ 9.11 (t, *J =* 1.7 Hz, 1H), 8.75 (d, *J* = 2.7 Hz, 1H), 8.35 (ddd, *J =* 9.9, 2.8, 1.8 Hz, 1H), 8.18 (s, 1H). |
| Example#8 | HPLC-MS A | 2.079 | 228.0 | ¹H NMR (599 MHz, dmso) δ 9.46 (d, *J* = 2.1 Hz, 1H), 9.06 (dt, *J* = 8.2, 1.7 Hz, 1H), 9.02 (dd, *J =* 5.7, 1.4 Hz, 1H), 8.13 (dd, *J* = 8.2, 5.6 Hz, 1H), 2.49 (s, 3H). |
| Example#9 | HPLC-MS A | 2.369 | 228.0 | ¹H NMR (400 MHz, CDCl₃) δ 9.44 (d, *J* = 2.4 Hz, 1H), 8.87 (dd, *J* = 4.8, 1.6 Hz, 1H), 8.50 (dt, *J =* 8.2, 2.0 Hz, 1H), 8.16 (s, 1H), 7.61 - 7.53 (m, 1H), 4.12 (s, 3H). |
| Example#10 | HPLC-MS A | 1.828 | 215.0 | ¹H NMR (400 MHz, MeOD) δ 9.86 (dd, *J* = 2.4, 1.2 Hz, 1H), 9.45 (dd, *J* = 5.4, 1.3 Hz, 1H), 8.34 (dd, *J* = 5.4, 2.4 Hz, 1H), 8.23 - 8.15 (m, 1H). |
| Example#11 | HPLC-MS A | 2.066 | 215.0 | ¹H NMR (400 MHz, MeOD) δ 9.41 (dd, *J* = 5.1, 1.6 Hz, 1H), 8.75 (dd, *J* = 8.6, 1.6 Hz, 1H), 8.28 (s, 1H), 8.00 (dd, *J* = 8.6, 5.0 Hz, 1H). |
| Example#12 | HPLC-MS C | 2.617 | 283.2 | ¹H NMR (400 MHz, MeOD) δ 8.68 (d, *J* = 1.6 Hz, 1H), 8.62 (dd, *J* = 9.6, 2.3 Hz, 1H), 8.21 (s, 1H), 7.26 (d, *J* = 9.7 Hz, 1H), 3.73 (d, *J* = 6.1 Hz, 4H), 2.27 - 2.16 (m, 4H). |
| Example#13 | HPLC-MS A | 1.724 | 214.0 | ¹H NMR (400 MHz, MeOD) δ 8.81 - 8.74 (m, 2H), 8.19 (s, 1H), 8.13 - 8.06 (m, 2H). |
| Example#14 | HPLC-MS A | 2.474 | 214.0 | ¹H NMR (400 MHz, MeOD) δ 8.78 (ddd, *J* = 4.8, 1.7, 0.9 Hz, 1H), 8.54 (dt, *J* = 8.0, 1.1 Hz, 1H), 8.23 (s, 1H), 8.06 (td, *J* = 7.8, 1.7 Hz, 1H), 7.63 (ddd, *J* = 7.6, 4.8, 1.2 Hz, 1H). |
| Example#15 | HPLC-MS C | 1.471 | 214.0 | ¹H NMR (400 MHz, DMSO-d6) δ 9.52 (d, J = 2.1 Hz, 1H), 9.14 (dt, J = 8.3, 1.8 Hz, 1H), 9.06 (dd, J = 5.7, 1.4 Hz, 1H), 8.26 (s, 1H), 8.18 (dd, J = 8.3 , 5.7 Hz, 1H). |
| Example#16 | HPLC-MS C | 3.402 | 349.0 | |
| Example#17 | HPLC-MS A | 2.102 | 220.0 | |
| Example#18 | HPLC-MS A | 2.076 | 221.0 | ¹H NMR (599 MHz, dmso) δ 9.91 (s, 1H), 8.22 (s, 1H). |
| Example#19 | HPLC-MS C | 1.834 | 203.0 | |
| Example#20 | HPLC-MS C | 1.942 | 220.0 | ¹H NMR (599 MHz, dmso) δ 8.23 (s, 1H), 8.11 - 8.06 (m, 2H). |
| Example#21 | HPLC-MS C | 2.140 | 219.0 | |
| Example#22 | HPLC-MS C | 1.795 | 217.0 | ¹H NMR (599 MHz, dmso) δ 8.13 (s, 1H), 7.86 (d, *J* = 2.3 Hz, 1H), 6.95 (d, *J* = 2.3 Hz, 1H), 3.94 (s, 3H). |
| Example#23 | HPLC-MS A | 2.095 | 217.0 | ¹H NMR (599 MHz, dmso) δ 8.51 (s, 1H), 8.21 (s, 1H), 8.12 (s, 1H), 3.89 (s, 3H). |
| Example#24 | HPLC-MS A | 1.851 & 1.954 | 203.0 | |
| Example#25 | HPLC-MS A | 1.184 | 203.0 | ¹H NMR (599 MHz, dmso) δ 9.30 (d, *J =* 1.3 Hz, 1H), 8.50 (d, *J* = 1.3 Hz, 1H), 8.33 (s, 1H). |
| Example#26 | HPLC-MS A | 1.960 | 203.0 | |
| Example#27 | HPLC-MS A | 1.729 | 253.0 | |
| Example#28 | HPLC-MS A | 3.031 | 264.0 | ¹H NMR (400 MHz, dmso) δ 10.25 (dd, *J* = 8.9, 1.4 Hz, 1H), 9.77 (s, 1H), 9.61 (s, 1H), 8.91 (s, 1H), 8.34 (dd, *J* = 8.4, 1.3 Hz, 1H), 8.19 - 7.98 (m, 2H). |
| Example#29 | HPLC-MS A | 2.161 | 230.0 | ¹H NMR (400 MHz, dmso) δ 8.75 (dd, *J* = 7.4, 2.2 Hz, 1H), 8.08 (s, 1H), 7.83 (dd, *J* = 6.2, 2.2 Hz, 1H), 6.61 (dd, *J* = 7.4, 6.2 Hz, 1H). |
| Example#30 | HPLC-MS C | 2.742 | 279.2 | |
| Example#31 | HPLC-MS A | 2.036 | 204.0 | ¹H NMR (400 MHz, dmso) δ 9.11 (d, *J* = 1.6 Hz, 1H), 8.22 (s, 1H), 7.13 (d, *J* = 1.8 Hz, 1H). |

## Claims

1. A compound having the following tautomeric structure of formula (I) 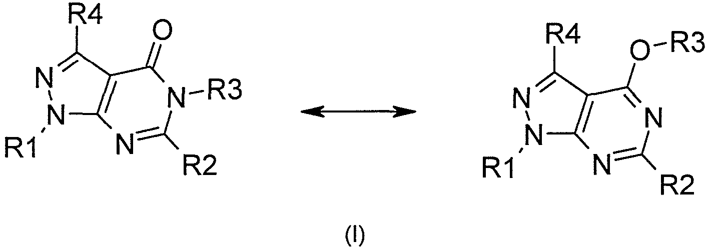 wherein
R1 is H or a C₁₋₆ alkyl group;
R2 is an optionally substituted phenyl group, an optionally substituted naphthyl group or an optionally substituted heteroaryl group containing 5 to 10 ring atoms selected from O, S, N and C;
R3 is H; and
R4 is H or a C₁₋₆ alkyl group;
or a salt thereof.

2. A compound according to claim 1, wherein R1 is H or a methyl group; especially wherein R1 is H.

3. A compound according to claim 1 or 2, wherein R4 is H or a methyl group; especially wherein R4 is H.

4. A compound according to claim 1, 2 or 3, wherein R2 is an optionally substituted phenyl group.

5. A compound according to claim 1, 2 or 3, wherein R2 is an optionally substituted heteroaryl group containing one ring and 5 or 6 ring atoms selected from O, S, N and C.

6. A compound according to claim 1, 2 or 3, wherein R2 is an optionally substituted pyridinyl group; especially wherein R2 is a 3-pyridinyl group.

7. A compound according to claim 1, 2 or 3, wherein R2 is an optionally substituted heteroaryl group containing two annellated rings and 9 or 10 ring atoms selected from O, S, N and C.

8. A compound according to claim 1 which is selected from the following compounds or a salt thereof:
6-(5-(trifluoromethyl)pyridin-3-yl)-1H-pyrazolo[3,4-d]pyrimidin-4(5H)-one
6-(6-methylpyridin-3-yl)-1H-pyrazolo[3,4-d]pyrimidin-4(5H)-one
6-(pyrazin-2-yl)-1H-pyrazolo[3,4-d]pyrimidin-4(5H)-one
6-(6-methoxypyridin-3-yl)-1H-pyrazolo[3,4-d]pyrimidin-4(5H)-one
6-(6-aminopyridin-3-yl)-1H-pyrazolo[3,4-d]pyrimidin-4(5H)-one
6-(6-(trifluoromethyl)pyridin-3-yl)-1H-pyrazolo[3,4-d]pyrimidin-4(5H)-one
6-(5-fluoropyridin-3-yl)-1H-pyrazolo[3,4-d]pyrimidin-4(5H)-one
3-methyl-6-(pyridin-3-yl)-2H-pyrazolo[3,4-d]pyrimidin-4(5H)-one
1-methyl-6-(pyridin-3-yl)-1H-pyrazolo[3,4-d]pyrimidin-4(2H)-one
6-(pyridazin-4-yl)-1H-pyrazolo[3,4-d]pyrimidin-4(5H)-one
6-(pyridazin-3-yl)-1H-pyrazolo[3,4-d]pyrimidin-4(5H)-one
6-(6-(pyrrolidin-1-yl)pyridin-3-yl)-1H-pyrazolo[3,4-d]pyrimidin-4(5H)-one
6-(pyridin-4-yl)-1H-pyrazolo[3,4-d]pyrimidin-4(5H)-one
6-(pyridin-2-yl)-1H-pyrazolo[3,4-d]pyrimidin-4(5H)-one
6-(pyridin-3-yl)-1H-pyrazolo[3,4-d]pyrimidin-4(5H)-one
6-[3,5-bis(trifluoromethyl)phenyl]-1H-pyrazolo[3,4-d]pyrimidin-4(5H)-one
6-(thiazol-5-yl)-1H-pyrazolo[3,4-d]pyrimidin-4(5H)-one
6-(1,2,3-thiadiazol-4-yl)-1H-pyrazolo[3,4-d]pyrimidin-4(5H)-one
6-(furan-3-yl)-1H-pyrazolo[3,4-d]pyrimidin-4(5H)-one
6-(thiazol-2-yl)-1H-pyrazolo[3,4-d]pyrimidin-4(5H)-one
6-(thiophen-3-yl)-1H-pyrazolo[3,4-d]pyrimidin-4(5H)-one
6-(1-methyl-1H-pyrazol-3-yl)-1H-pyrazolo[3,4-d]pyrimidin-4(5H)-one
6-(1-methyl-1H-pyrazol-4-yl)-1H-pyrazolo[3,4-d]pyrimidin-4(5H)-one
6-(1H-pyrazol-4-yl)-1H-pyrazolo[3,4-d]pyrimidin-4(5H)-one
6-(1H-imidazol-4-yl)-1H-pyrazolo[3,4-d]pyrimidin-4(5H)-one
6-(1H-pyrazol-3-yl)-1H-pyrazolo[3,4-d]pyrimidin-4(5H)-one
6-(4H-pyrrolo[3,2-b]pyridin-6-yl)-1H-pyrazolo[3,4-d]pyrimidin-4(5H)-one
6-(quinolin-3-yl)-1H-pyrazolo[3,4-d]pyrimidin-4(5H)-one
6-(2-oxo-1,2-dihydropyridin-3-yl)-2H-pyrazolo[3,4-d]pyrimidin-4(5H)-one
6-(3-methylquinoxalin-2-yl)-2H-pyrazolo[3,4-d]pyrimidin-4(5H)-one
6-(isoxazol-3-yl)-1H-pyrazolo[3,4-d]pyrimidin-4(5H)-one.

9. A pharmaceutical composition comprising a compound according to any one of the preceding claims and optionally one or more carrier substances and/or one or more adjuvants.

10. A compound according to any one of claims 1 to 8 or a pharmaceutical composition according to claim 9 for use in the treatment of diseases, disorders, and conditions that are associated with aberrant activity of one or more aquaporins.

11. The compound or the pharmaceutical composition for use according to claim 10 wherein the aquaporin is AQP4.
